# EUROPÄISCHE PATENTANMELDUNG

(11) **EP 1 300 202 A1**
(43) Veröffentlichungstag der Anmeldung: **09.04.2003**
(21) Anmeldenummer: 02021048.0
(22) Anmeldetag: 20.09.2002
(51) Int. Cl.: B09B 3/00, B02C 19/12, A61L 11/00

(54) **Vorrichtung zur Behandlung medizinischer Abfälle**

(30) Priorität: 08.10.2001 DE 20116476 U
(71) Anmelder: Salda, Luciano, 41058 Vignola (MO) (IT)
(72) Erfinder: Salda, Luciano, 41058 Vignola (MO) (IT)
(74) Vertreter: Grünecker, Kinkeldey, Stockmair & Schwanhäusser Anwaltssozietät

(57) **Zusammenfassung**

Die Erfindung betrifft eine Vorrichtung (1) zum Bearbeiten von medizinischem Abfall, mit einem Eingaberaum (2), einem Förderer zum Transport des Abfalls (8) zu einem Verdichter (11), mindestens einer sterilisierenden Bearbeitungseinrichtung (21) und einem Auslass (23) zum Auslassen des sterilisierten Abfalls. Die Vorrichtung zeichnet sich dadurch aus, dass sie eine verfahrbare Absperrhülse (26, 32) zwischen dem Förderer (8) und dem Verdichter (11) aufweist.

## Beschreibung

Die Erfindung betrifft eine Vorrichtung zum Bearbeiten, insbesondere Komprimieren und Sterilisieren, von medizinischem Abfall. Diese Vorrichtung besitzt einen Eingaberaum, einen Förderer zum Transport des Abfalls zu einem Verdichter, mindestens eine sterilisierende Bearbeitungseinrichtung und einen Auslass zum Auslassen des sterilisierten Abfalls.

Der in Krankenhäusern, Arztpraxen oder ähnlichen medizinischen Einrichtungen erzeugte Abfall weist in der Regel stark infektiöse Anteile auf und muss daher insgesamt als Sondermüll behandelt werden. Erst eine Sterilisierung ermöglicht eine kostengünstige und gefahrlose Entsorgung des medizinischen Abfalls zusammen mit übrigen Abfällen auf einer Deponie. Vorrichtungen für solch eine Sterilisierung von infektiösen medizinischen Abfällen sind aus der Praxis bekannt. Die Sterilisierung kann dabei auf verschiedenen Wegen durchgeführt werden: z.B. rein thermisch (durch Erhitzen auf 200°C oder mehr, meist über mehrere Minuten), chemisch, insbesondere nass-chemisch, oder durch Bestrahlung mittels Ultraviolett-Licht (UV) oder Mikrowellen. Letzteres vermeidet eine chemische Belastung des Abfalls und gewährleistet im Gegensatz zum oberflächlichen Erhitzen oder UV-Bestrahlen eine gleichmäßige Erhitzung des Abfalls auch im Innern.

Es ist vorteilhaft, den Abfalls bereits vor dem Erreichen der Sterilisiereinrichtung nicht nur zu zerkleinern, sondern auch stark zu verdichten, um den Raumbedarf für die Sterilisiereinrichtung und den letztendlich zu entsorgenden Abfall zu verringern. Dabei ergibt sich jedoch eine Schwierigkeit durch die sehr unterschiedlich hohen Flüssigkeits-Anteile im Abfall: die Bearbeitungsvorrichtung muss einen durchgehenden Bearbeitungspfad für den Abfall bieten, gleichzeitig jedoch eine Rückwärts-Bewegung des Abfalls entlang dieses Pfades insbesondere bei einem Verdichtungsvorgang verhindern.

Eine Bearbeitungsanlage für infektiösen, medizinischen Abfall, die eine definierte und stetige Richtung des Abfallstroms hat, ist beispielsweise aus der Europäischen Patentschrift 0 908 189 "Processing facility for disposing of infectious medical waste" bekannt. In dieser Vorrichtung durchläuft der infektiöse Abfall einen Verarbeitungspfad, entlang dessen er zunächst in möglichst kleine Bestandteile zerkleinert und anschließend für eine genügend lange Zeit, typischerweise bis zu einer Stunde, durch Kontakt mit einer erhitzten Oberfläche ultrahocherhitzt wird. Dieser Kontakt findet bevorzugt in einem nebeneinanderliegenden Paar von Schnecken statt, die den Abfall entlang des Verarbeitungspfades transportieren.

Zwar ist auch in diesem Stand der Technik eine Abdichtung des Innern der Vorrichtung vorgesehen. Da die Verdichtung des Abfalls nach jener Lehre jedoch erst außerhalb der Bearbeitungsvorrichtung erfolgt, steht beim Abdichten des Vorrichtungsinnern im Vordergrund, einen Austausch von infektiösen Keimen über die Luft zu verhindern. Folglich sind die Abdichtungen als reine Luftschleusen ausgestaltet, deren Zweck es ist, den Abfallstrom passieren zu lassen, den Austausch von Luft aber zu unterbinden.

Es ist daher eine Aufgabe der vorliegenden Erfindung, eine Vorrichtung zum Bearbeiten von medizinischem Abfall zur Verfügung zu stellen, die unabhängig von der Zusammensetzung des Abfalls, insbesondere in Bezug auf flüssige Bestandteile, eine Verdichtung des Abfalls bereits in ihrem Innern gestattet, ohne dass diese Verdichtung zu einer ungewollten Rückwärtsbewegung des Abfalls in der Vorrichtung führt.

Diese und weitere Aufgaben werden erfindungsgemäß in einer Vorrichtung nach dem Oberbegriff des Anspruchs 1 dadurch gelöst, dass sie eine verfahrbare Absperrhülse zwischen dem Förderer und dem Verdichter aufweist.

Diese Absperrhülse dient dazu, während einer Verdichtung des Abfalls, während des Bearbeitens und bis zum Auslassen des sterilisierten Abfalls den Verdichter gegenüber dem ihn beliefernden Verarbeitungspfad, insbesondere dem Förderer, abzusperren. Diese Absperrung muss dabei die Bedingung erfüllen, dass sie trotz der während des Verdichtens aufgebauten Drücke keinen Fluss von Abfall in rückwärtiger Richtung gestattet.

Erfindungsgemäß kann die Absperrhülse auf unterschiedliche Arten ausgebildet sein, wobei sie bevorzugt im Verbindungsbereich zwischen dem Förderer und dem Verdichter angeordnet ist. Während in einer Ausführungsform die Absperrhülse konstruktiv eher dem Förderer zugeordnet ist, ist sie in einer anderen Ausführungsform konstruktiv eher dem Verdichter zugeordnet. Diese Ausführungsformen sind in den Figuren für zwei Ausführungsbeispiele konkret dargestellt.

Vorteilhaft ist es, wenn die Absperrung neben einer mechanischen Absperrung überwiegend für feste Abfallkomponenten auch eine hermetische Abdichtung bietet, d.h. eine wasser- und luftdichte Abdichtung für flüssige oder in Luft gelöste Komponenten des Abfalls- und wenn auch diese hermetische Abdichtung angesichts der hohen Drücke in der Vorrichtung aufrechterhalten werden kann. Mögliche Ausführungsformen solch einer Abdichtung werden im Folgenden noch genauer erläutert.

Eine Eingabeeinheit über dem Eingaberaum erleichtert das Einfüllen des Abfalls in die Vorrichtung und verhindert ein Überlaufen des Eingaberaums. Diese Eingabeeinheit kann beispielsweise als ein in Richtung des Förderers langgestreckter Einfülltrichter ausgebildet sein.

Eine Zerkleinerungseinrichtung über der Eingabeeinheit lässt den zerkleinerten Abfall in den Einfülltrichter hineinfallen. Diese Zerkleinerung erleichtert sowohl die nachfolgende Aufnahme des Abfalls durch den Förderer, als auch die effektive Verdichtung des Abfalls.

In der Eingabeeinheit oder im Eingaberaum, bevorzugt etwas oberhalb des Förderers, kann sich ein Sensor befinden, der die Anwesenheit von Abfall registriert. Dieser kann beispielsweise als Lichtschranke ausgebildet sein. Von Vorteil ist dabei, wenn über den Sensor festgestellt werden kann, wie lange der Durchgang des letzten Abfalls zurückliegt. Dies kann z.B. über eine geeignete Elektronik ermöglicht werden.

Der Förderer ist bevorzugt als Stetigförderer ausgebildet, um Totzeiten beim Transport des Abfalls aus dem Eingaberaum zu minimieren. Insbesondere kann dafür eine Schnecke gewählt werden. Bevorzugt ist die Schnecke fliegend gelagert, mit dem Antrieb an ihrem Anfang. Ihr Ende kann damit frei liegen, um den Übergang des Abfalls vom Eingaberaum zum Verdichter bzw. zum Bearbeitungsraum nicht zu behindern. Idealerweise liegt die Schnecke mit ihren Windungen dicht am Boden des Eingaberaums an, um den dort angesammelten Abfall vollständig mitnehmen zu können. Indem sie durch den Eingaberaum hindurchläuft, erfasst sie den in diesem enthaltenen Abfall und zieht ihn aktiv in ihre Windungen hinein So bewirkt sie eine leichte Vor-Verdichtung des Abfalls.

Die Richtung des Förderers weist in der Regel zum Verdichter. In einer besonders vorteilhaften Ausführungsform der Erfindung ist jedoch der Antrieb des Förderers umschaltbar, seine Förderrichtung kann also umgekehrt werden. Dadurch wird der am Ende des Förderers liegende Abfall aus dem Verbindungsbereich zum Verdichter zurücktransportiert. So wird ohne zusätzliche mechanische Einrichtungen wirksam verhindert, dass der Abfall während eines Verdichtungshubes des Verdichters in diesen hineinfällt. Von Vorteil ist dabei, wenn der Anfangsbereich des Förderers nicht vom Einfülltrichter überdeckt wird und sich noch vor dem Eingaberaum befindet. Damit wird erreicht, dass der Anfangsbereich des Förderers frei von Abfall bleibt, so dass dort bei der Umkehr der Antriebsrichtung keine Verdichtung des Abfalls erfolgt, die möglicherweise eine höhere Antriebsleistung verlangen würde. Notwendig dafür ist, dass eine ausreichend lange Strecke am Anfang des Förderers außerhalb des Eingaberaums liegt. Beispielsweise kann diese Strecke einer halben Ganghöhe der Schnecke entsprechen, bevorzugt jedoch mindestens einer vollen Ganghöhe.

Von Vorteil ist es, im Eingaberaum und/oder im Förderer, insbesondere in dessen Anfangsbereich, eine oder mehrere Vorrichtungen zum Besprühen des Abfall mit einer Desinfektionslösung vorzusehen. Dies verhindert ein Infektionsrisiko durch im Eingaberaum befindlichen Abfall, insbesondere bei einem Ausfall der Vorrichtung oder ihrer Abschaltung. Zudem unterstützt dies die anschließende Sterilisierung.

Der Verdichter, der mit dem Förderer über eine Austrittsöffnung des Förderers verbunden ist, ist bevorzugt als ein unstetiger Verdichter ausgebildet. Damit hat er den Vorteil, gegenüber Stetigverdichtern eine deutlich stärkere Verdichtung zu ermöglichen. Eine solche Verdichtung bietet insgesamt den Vorteil eines geringeren Raumbedarfs für die Sterilisiereinrichtung sowie eines geringeren zu entsorgenden Abfallvolumens. Dadurch wiederum werden die Kosten für die Bearbeitungsanlage, den Transport des Abfalls zur Deponie und seine dortige Deponierung gesenkt.

Indem der Verdichter als Förderer konstruiert wird, kann er zwei zusätzliche Funktionen erfüllen:
a) die Förderung des Abfalls hin zu einem Bearbeitungsraum, der sich am Ende des Verdichters befindet und in dem der verdichtete Abfall mittels der sterilisierenden Bearbeitungseinrichtung sterilisiert wird, sowie
b) den Ausstoß des sterilisierten Abfalls aus der Vorrichtung im Anschluss an die Bearbeitung.

Für eine besonders starke Verdichtung kann der Verdichter z.B. einen in einem Zylinder geführten Verdichterkolben aufweisen, der eine Bereitschaftsposition und eine Verdichtungsposition besitzt und zwischen den beiden Positionen einen Verdichtungshub ausführen kann. Zusätzlich kann er eine Endposition besitzen, zu der er einen zusätzlichen Hub zum Ausstoß des Abfalls aus der Vorrichtung durchführen kann, dieser zusätzliche Hub entspricht dabei vorteilhafterweise etwa der Höhe des Bearbeitungsraumes. Daher wird dieser Kolben im Folgenden als Ausstoß-Verdichterkolben bezeichnet.

Die Achse des Zylinders des Verdichters ist dabei bevorzugt, aber nicht zwingend, vertikal angeordnet. Der Ausstoß-Verdichterkolben kann am oberen Ende des Zylinders so gelagert sein, dass er in seiner Bereitschaftsposition oberhalb der Austrittsöffnung des ersten Verdichters liegt. So wird erreicht, dass der Abfall beim Übergang vom Förderer in den Verdichter einem freien Fall ausgesetzt wird, in dem sich eine eventuell durch den Förderer hervorgerufene Orientierung auflösen kann. Zusätzlich wirkt bei dieser Anordnung die Gravitationskraft in der gleichen Richtung wie die Verdichtung, unterstützt diese also; und sie hält den dann verdichteten Abfall im Bearbeitungsraum am unteren Abschnitt des Verdichters. Dies ist insbesondere dann von Vorteil, wenn der Flüssigkeitsanteil im Abfall hoch ist; denn die Gravitation verhindert so einen Rückfluss des Abfalls aus dem Bearbeitungsraum.

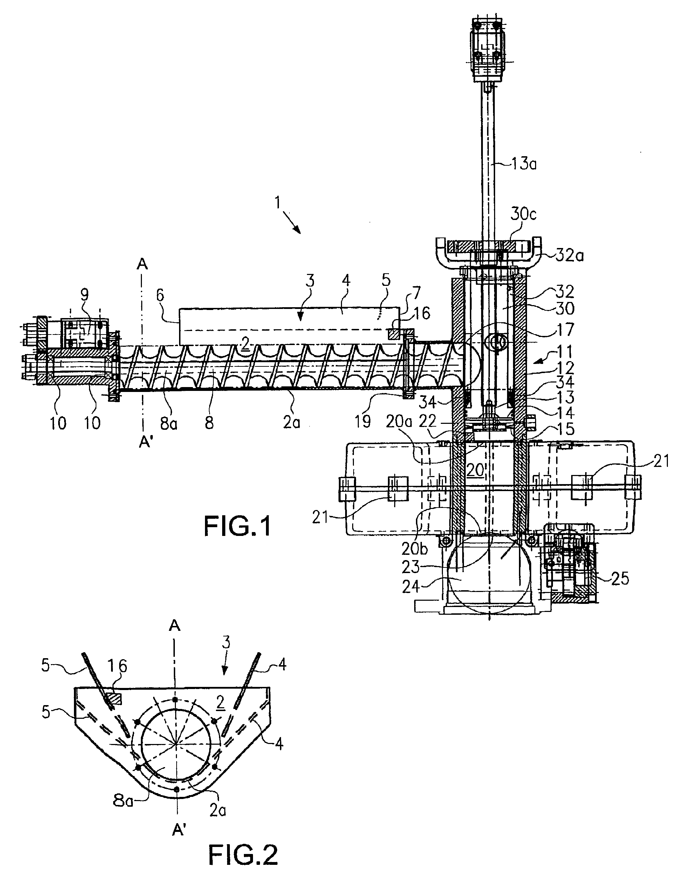

Der Ausstoß-Verdichterkolben kann eine Dichtlippe für seine Abdichtung gegenüber dem Zylinder aufweisen, sowie eine Bürste und/oder Metallschaber, die in Förderrichtung vor der Dichtlippe liegen und dafür sorgen, dass beim Verdichtungsvorgang keine Abfallreste an der Innenwand des Zylinders hängen bleiben. Ein besonders vorteilhaftes Ausführungsbeispiel des Verdichters ist in den Figuren 1, 3 und 4 dargestellt; es weist drei relativ zueinander und relativ zum Zylinder bewegbare Elemente auf: einen Ausstoß-Verdichterkolben, einen Zylinderkolben und eine Absperrhülse, deren jeweilige Funktionen bei der Beschreibung des Ausführungsbeispiels im Einzelnen erläutert werden.

Für die Ausbildung der verfahrbare Absperrhülse zwischen dem Förderer und dem Verdichter ergeben sich bei einer Vorrichtung mit den bisher beschriebenen Merkmalen mindestens zwei unterschiedliche Varianten. In einer Ausführungsform A) ist die Absperrhülse konstruktiv eher dem Förderer zugeordnet ist, während sie in einer anderen Ausführungsform B) konstruktiv eher dem Verdichter zugeordnet ist:
A) Zum Beispiel kann die Absperrhülse als Schiebehülse ausgebildet sein, die im Gehäuse des Förderers gegenüber diesem verfahrbar ist. Diese Schiebehülse besitzt mindestens zwei verschiedene Stellungen, eine Durchgangs- und eine Sperrstellung, sowie eine Durchgangsöffnung, die sich vorteilhafterweise auf ihrer Unterseite befindet. Ihre Durchgangsstellung nimmt die Schiebehülse dann ein, wenn sich der Ausstoß-Verdichterkolben an seiner Bereitschaftsposition befindet. Dabei ragt sie so weit in den Verdichter hinein, dass die Durchgangsöffnung auf ihrer Unterseite frei ist und der vom Förderer herangeführte Abfall durch diese Öffnung in den Verdichter hinein fallen kann. Vor einem Verdichtungshub des Verdichters jedoch wird die Schiebehülse gegenüber dem Förderer verschoben, so dass sie nicht mehr in den Verdichter hineinragt und die Öffnung auf ihrer Unterseite verschlossen ist. Zweckmäßigerweise ist dazu vorher der Antrieb des Förderers gestoppt worden, um eine weitere Förderung des Abfalls zu unterbinden, oder sogar ggf. umgekehrt worden (s.o.), damit das Einfahren der Schiebehülse nicht gegen den Widerstand von am Ende des Förderers vorliegendem Abfall durchgeführt werden muss. Dazu ist es von Vorteil, wenn die Strecke vom Anfang des Förderers bis zum Eingaberaum mindestens so lang ist wie die Strecke, um die die Schiebehülse verschoben wird, da dann der Abfall bei einem Rücktransport nicht verdichtet werden muss. Erst wenn sichergestellt ist, dass die Schiebehülse eine freie Bewegung des Verdichterkolbens nicht länger behindert, und sie ggf. noch mittels eines geeigneten Mechanismus in ihrer Sperrstellung verriegelt worden ist, setzt der Verdichtungshub des Kolbens ein.
   Der Änderung der Bewegungsrichtung des Abfalls vom Förderer zum Verdichter, die innerhalb der Schiebehülse stattfindet, kann in dieser Ausführungsform auf zweierlei Weisen Rechnung getragen werden: Zum einen kann innerhalb der Schiebehülse ein Umlenker vorgesehen werden, z.B. ein schräger Keil, zum anderen kann die Schiebehülse in ihrer Durchgangsstellung abgestützt werden, um Rückstoßkräfte abzulenken. Solch eine Abstützung kann beispielsweise dadurch gebildet werden, dass die Schiebehülse in eine der Austrittsöffnung gegenüberliegende Vertiefung in der Wand des Verdichters eingefahren wird, in der sich die Schiebehülse ausschließlich axial, also in ihrer Verfahrrichtung, bewegen kann.
B) Die Absperrhülse kann alternativ aus einer Zylinderhülse im Verdichter, evtl. zusätzlich mit einem Dichtungspaket zwischen ihr und einem Verdichterkolben, gebildet sein. Wenn diese Zylinderhülse vor dem Verdichtungshub nach unten fährt, kann sie gleichzeitig die Austrittsöffnung des Förderers schließen und das Dichtungspaket beaufschlagen, so dass dieses den am Ende des Verdichters liegenden Bearbeitungsraum wirkungsvoll hermetisch abdichtet.

Vorteilhaft ist eine Ausführungsform der Vorrichtung, bei der ein Sensor im Eingaberaum und/oder die Antriebe des Förderers und/oder des Verdichters und/oder der Absperrhülse miteinander koordiniert sind. Dadurch kann z.B. erreicht werden, dass der Förderer gestoppt wird, sobald eine ausreichend lange Zeitspanne seit der letzten Registrierung von Abfall durch den Sensor verstrichen ist. "Ausreichend lange" bedeutet dabei, dass der Förderer mit hoher Wahrscheinlichkeit frei von Abfall ist. Die zum Transport einer Abfallkomponente durch den Förderer benötigte Zeit ergibt sich durch dessen Fördergeschwindigkeit. Insbesondere ist es mittels einer Koordination der Antriebe des Förderers, des Verdichters und der Absperrhülse möglich, unmittelbar vor einem Verdichtungshub des Verdichters den Antrieb des Förderers zu stoppen oder ggf. rückwärts laufen zu lassen und die Absperrhülse zwischen dem Förderer und dem Verdichter in ihre Sperrstellung zu bringen. Solch eine Koordinierung kann z.B. mittels eines Steuerprogramms auf einem Mikrochip oder extern mittels eines Rechners durchgeführt werden.

Vorteilhaft ist es ferner, wenn der Hub des Ausstoß-Verdichterkolbens in etwa am Einlass des Bearbeitungsraumes gestoppt und der Kolben in dieser Position gehalten werden kann. So ist gesichert, dass sich nach der Verdichtung der gesamte Abfall im Innern des Bearbeitungsraumes befindet und somit einer sterilisierenden Bearbeitung zugänglich ist. Andernfalls, d.h. bei zu kurzem Hub des Kolbens, würde riskiert, dass ein Teil des Abfalls die Vorrichtung unsterilisiert verlässt.

Diese Anordnung bietet zudem den großen Vorteil, dass der Kolben nach der Verdichtung während der Bearbeitung des Abfalls in dieser Position verbleiben und so einen mechanischen Verschluss des Bearbeitungsraums gegenüber dem restlichen Abschnitt des Verdichters darstellen kann, ohne dass eine zusätzliche Einrichtung wie z.B. eine Tür oder eine Klappe dazu notwendig wären, an deren beweglichen Teilen Abfallreste hängenbleiben könnten.

Am Einlass des Bearbeitungsraumes oder etwas oberhalb davon kann sich im Innern des Verdichters ein Sensor befinden, der die Anwesenheit von Abfall auf seiner Höhe innerhalb des Verdichters registriert. Darüber kann der Antrieb des Verdichters so gesteuert werden, dass ein Verdichtungshub erst dann durchgeführt wird, wenn Abfall bis zu dieser Höhe in dem Verdichter und damit im Bearbeitungsraum vorliegt. Ferner kann eine Verdichtung so lange durchgeführt werden, bis durch den Sensor sichergestellt ist, dass sich kein Abfall mehr außerhalb des Bearbeitungsraumes befindet. Dadurch wird gewährleistet, dass der gesamte Abfall der sterilisierenden Bearbeitung ausgesetzt ist.
Diese Funktionen können auch durch mehrere Sensoren auf verschiedenen Höhen des Zylinders wahrgenommen werden.

Bei der erfindungsgemäßen Vorrichtung ist ein Mikrowellenstrahler in der Bearbeitungseinrichtung vorgesehen. Dieser ermöglicht eine Erhitzung des gesamten, komprimierten Abfallvolumens auch in dessen Innern. Für eine ausreichende Sterilisierung des gesamten Abfalls wird die Erhitzung über einen Zeitraum von einer bestimmten Dauer durchgeführt. Diese Zeitdauer kann - gerade im Vergleich zu bekannten Vorrichtungen - sehr kurz sein, da die benötigten Drücke und Temperaturen innerhalb von etwa einer Minute erreicht werden und der im Bearbeitungsraum enthaltene Abfall innerhalb von 10-15 Sekunden sterilisiert werden kann. Mittels der Mikrowellenstrahlung kann auch der in den Bearbeitungsraum hineinragende Teil des Verdichters an der Sterilisierung teilnehmen.

Zweckmäßigerweise besitzt der Auslass der Vorrichtung, insbesondere also des Bearbeitungsraumes, einen Verschluss, der z.B. als ein Kugel- oder ein Schiebeventil ausgebildet sein kann und einen eigenen Antrieb aufweisen kann. Dieser Verschluss gewährleistet, dass der gesamte Abfall bei der Verdichtung und der nachfolgenden Bearbeitung im Bearbeitungsraum verbleibt und die Vorrichtung erst in sterilisiertem Zustand wieder verlässt. Dem Verdichtungsdruck muss der Verschluss dabei einen genügend hohen Widerstand entgegensetzen können.

Von Vorteil ist es, wenn der Ausstoß-Verdichterkolben von seiner Verdichtungs-Stellung am Einlass 20a des Bearbeitungsraums aus einen zusätzlichen Hub nach unten um insgesamt die Höhe des Bearbeitungsraums 20 ausführen kann, d.h. in seine Endposition. So kann der Kolben nach Öffnen des Auslasses dazu dienen, den sterilisierten Abfall aus der Vorrichtung herauszuschieben. Dazu ist es vorteilhaft, wenn sich der Kolben zumindest teilweise während der Sterilisierung im Bearbeitungsraum befindet, da er so beim Ausschieben des Abfalls keine erneute Kontaminierung des Abfalls verursacht.

Die Erfüllung dieser und weiterer Vorteile ergeben sich aus der Beschreibung der zwei Ausführungsbeispiele, die im Folgenden anhand einer Zeichnung dargestellt werden. Es zeigen:
- Fig. 1: einen Vertikalschnitt durch ein erstes Ausführungsbeispiel einer erfindungsgemäßen Vorrichtung zur Bearbeitung von medizinischem Abfall;
- Fig. 2: einen Vertikalschnitt durch den Förderer in dessen Anfangsbereich an der in Fig. 1 mit AA' bezeichneten Stelle;
- Fig. 3: Vertikalschnitt durch den Verdichter des ersten Ausführungsbeispiels, in vergrößertem Maßstab;
- Fig. 4 a)-c): Vertikalschnitte durch den Verdichter des ersten Ausführungsbeispiels in drei verschiedenen Positionen während eines Verdichtungsvorgangs: a) nach dem ersten Verdichtungshub des Ausstoß-Verdichterkolbens, b) nach einem Haupt-Verdichtungshub, c) nach dem Ausstoß des sterilisierten Abfalls mittels des Ausstoß-Verdichterkolbens;
- Fig. 5: einen Vertikalschnitt durch einen Ausschnitt eines zweiten Ausführungsbeispiels einer erfindungsgemäßen Vorrichtung mit einer Schiebehülse als Absperrung, speziell mit der Schiebehülse in ihrer Durchgangsstellung;
- Fig. 6: denselben Ausschnitt wie in Fig. 5, speziell mit der Schiebehülse in ihrer Sperrstellung.

In Fig. 1 ist ein Vertikalschnitt durch ein erstes Ausführungsbeispiel einer erfindungsgemäßen Vorrichtung 1 zur Bearbeitung von medizinischem Abfall dargestellt. Sie besitzt einen langgestreckten Eingaberaum 2 und eine Eingabeeinheit 3, die in diesem Beispiel in Form eines Einfülltrichters ausgebildet ist, der sich vom Eingaberaum 2 nach oben öffnet. Der Einfülltrichter 3 besitzt zwei Seitenwände 4,5, die in Längsrichtung des Eingaberaums 2 liegen und in ihrem Querschnitt in Fig. 2 gezeigt sind. Diese Fig. 2 stellt einen Schnitt an der Stelle AA' dar. Der Einfülltrichter besitzt ferner zwei lotrechte Stirnwände 6,7.

Der von den Seitenwänden 4,5 gebildete Öffnungswinkel verändert sich in der Längsrichtung des Einfülltrichters; insbesondere verringert er sich in der Förderrichtung des ersten Verdichters 8 von etwa 90° am Anfang, d.h. an der Stirnwand 6, auf etwa 45° am Ende, d.h. an der Stirnwand 7. Die vier Wände 4-7 des Einfülltrichters 3 bilden in diesem Ausführungsbeispiel gleichzeitig die Wände des Eingaberaums 2.

Oberhalb der Eingabeeinheit 3 ist eine hier nicht gezeigte Zerkleinerungseinrichtung angeordnet, durch die der zerkleinerte Abfall in den Einfülltrichter hineinfällt.

An der Unterseite des Eingaberaums 2 befindet sich ein Förderer 8, der in diesem Beispiel als eine Schnecke ausgebildet ist. Die Schnecke 8 erstreckt sich ebenfalls in Längsrichtung des Eingaberaums 2, wobei der Boden 2a des Eingaberaums 2 so geformt ist, dass er - wie in Fig. 2 ersichtlich - über einen gewissen Bereich bündig an der Schnecke 8 anliegt. Die Schnecke 8 hat einen Antrieb 9, an dem sie mittels eines Lagers 10 fliegend gelagert ist, sowie einen Anfangsbereich 8a auf der Seite dieser Lagerung 10, wobei der Anfangsbereich 8a außerhalb des Eingaberaums 2 und damit vor dem Einfülltrichter 3 liegt.

Zwischen der Oberseite des Eingaberaums 2 und der Oberseite des Förderers 8 befindet sich im Eingaberaum 2 ein Sensor 16, der das Vorhandensein oder den Durchgang von Abfall auf seiner Höhe registriert und mit einer geeigneten Auswerteeinheit verbunden ist (nicht gezeigt), die die Bestimmung der seit der letzten Registrierung von Abfall verstrichenen Zeitspanne erlaubt. Dieser Sensor weist z.B. eine Lichtschranke auf.

Im Normalbetrieb nimmt die Schnecke 8 den Abfall aus dem Eingaberaum 2 bzw. von dessen Boden 2a auf, und befördert ihn dann in Richtung des Verdichters 11.

Der Antrieb 9 der Schnecke ist umschaltbar. Dadurch kann die Schnecke vor einem Verdichtungshub des Verdichters 11 um eine gewisse Strecke zurückgefahren werden, bis der Bereich vor der Austrittsöffnung 17 frei von Abfall ist. So kann verhindert werden, dass Abfall während eines Verdichtungshubes des Verdichters 11 aus der Öffnung 17 in diesen hinein fällt. Da der Anfangsbereich 8a des Förderers 8 nicht vom Einfülltrichter überdeckt wird und sich noch vor dem Eingaberaum 2 befindet, wird erreicht, dass dieser Anfangsbereich 8a frei von Abfall bleibt, so dass dort bei der Umkehr der Antriebsrichtung keine Verdichtung des Abfalls erfolgt. In dem in Fig. 1 gezeigten Ausführungsbeispiel hat der Anfangsbereich 8a eine Länge von etwa zweieinhalb Ganghöhen der Schnecke 8.

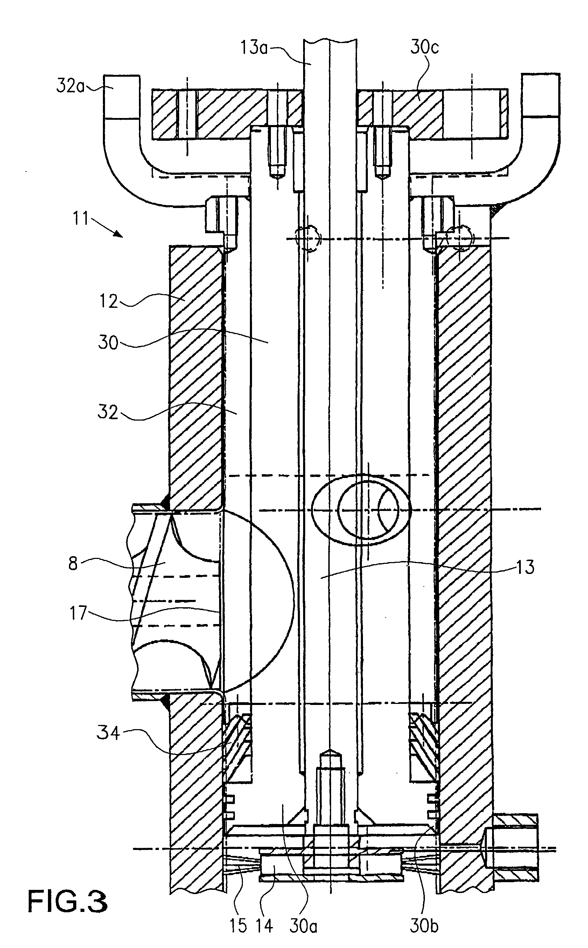

Der Verdichter 11 und einige seiner Elemente sind zum besseren Verständnis in der Figur 3 noch einmal vergrößert dargestellt. Der Verdichter 11 weist einen unbeweglichen, vertikalen, äußeren Zylindermantel 12 auf, dessen Wand an einer Seite in ihrem oberen Abschnitt die Austrittsöffnung 17 der Fördereinrichtung 8 aufweist. Im Innenraum des Zylindermantels 12 sind drei zylinderförmige Elemente axial und damit vertikal bewegbar: als innerstes Element ein auf der Zylinderachse laufender Ausstoß-Verdichterkolben 13, der umfasst ist von einem hohlen Zylinderkolben 30. Eine als ebenfalls hohle Zylinderhülse 32 ausgebildete Absperrhülse umfasst wiederum den Zylinderkolben 30 und ist bewegbar zwischen dem Zylinderkolben 30 und dem äußeren Zylindermantel 12. Die drei Elemente 13, 30 und 32 weisen jeweils einen eigenen Antrieb auf, der sie in vertikaler Richtung bewegt. Die Antriebe sind außerhalb des unbeweglichen Zylindermantels 12 angeordnet und in den Figuren nicht dargestellt.

Der Ausstoß-Verdichterkolben 13 weist einen Kolbenschaft 13a auf, dessen Länge mindestens so groß ist wie die Höhe des Zylindermantels 12 des Verdichters 11. Am unteren Ende des Kolbenschafts 13a ist ein Verdichtungsteller 14 angebracht, der einen etwas kleineren Durchmesser als der Querschnitt des Zylindermantels 12 hat. An der Peripherie dieses Verdichtungstellers 14 wiederum ist eine dichte, ringförmige Bürste 15 angeordnet, deren Borsten sich radial vom Verdichtungsteller 14 bis zur Innenwand des Zylindermantels 12 erstrecken.

Der Ausstoß-Verdichterkolben 13 dient dazu, zunächst ohne den Zylinderkolben 30 und die Zylinderhülse 32 einen ersten Verdichtungshub auf den im Bearbeitungsraum angesammelten Abfall durchzuführen. Bei dieser Hubbewegung reinigt die in Förderrichtung vor dem Verdichtungsteller 14 liegende Bürste 15 den Innenraum des Verdichters von Abfall und führt diesen Abfall mit in den Bearbeitungsraum 20.

Der hohle Zylinderkolben 30 nimmt in seinem Innern den Ausstoß-Verdichterkolben 13 auf, speziell dessen Kolbenschaft 13a. Sein Außendurchmesser ist über den Großteil seiner Länge kleiner als der Innendurchmesser des Zylindermantels 12. Lediglich der untere Abschnitt 30a des Zylinderkolbens 30 entspricht mit seinem Durchmesser dem Zylindermantel 12 und wird von diesem eng, aber bewegbar aufgenommen. Am Rand des Abschnitts 30a sind auf seiner Unterseite Metallschaber 30b vorgesehen, die an der Innenwand des Zylindermantels 12 anliegen. Sie dienen zum Säubern des Zylindermantels 12, insbesondere der Austrittsöffnung 17, von Abfall, der beim ersten Verdichtungshub nicht von der Bürste 15 erfasst wurde.

Die äußerste der drei im Zylindermantel 12 beweglichen Komponenten ist die Zylinderhülse 32. Sie liegt eng, aber bewegbar zwischen dem Zylinderkolben 30 und der Innenwand des Zylindermantels 12. Die Länge der Zylinderhülse 32 entspricht mindestens der Distanz vom oberen Ende des Zylindermantels 12 bis zum tiefsten Punkt der Austrittsöffnung 17 der Fördereinrichtung 8. In diesem Ausführungsbeispiel ist die Absperrhülse demnach konstruktiv eher dem Verdichter zugeordnet.

In der Bereitschaftsposition der drei beweglichen Elemente des Verdichters 11 befindet sich die Zylinderhülse 32 vollständig oberhalb der Austrittsöffnung 17, behindert also den Durchgang von Abfall durch die Austrittsöffnung 17 nicht. Während des Haupt-Verdichtungshubes jedoch fährt sie gemeinsam mit dem Ausstoß-Verdichterkolben 13 und dem Zylinderkolben 30 nach unten und erfüllt dabei zwei Funktionen: zum einen sperrt sie die Austrittsöffnung 17 gegenüber dem Verdichter 11 mechanisch ab und verhindert so, dass weiterer Abfall in den Verdichter 11 hineingelangt. Vor allem jedoch übt sie Druck auf ein auf der Oberseite des verbreiterten Abschnitts 30a des Zylinderkolbens 30 liegendes Dichtungspaket 34 auf. Dieses Dichtungspaket 34 befindet sich ringförmig auf der Oberseite des Abschnitts 30a und besteht aus elastischem Kunststoff, der durch den Druck der Zylinderhülse 32 eine hermetische Abdichtung des unteren Abschnitts des Verdichters 11, damit insbesondere des Bearbeitungsraums 20, vom oberen Abschnitt des Verdichters erzielt.

Die Zylinderhülse 32 weist an ihrem oberen Ende außerhalb des Zylindermantels 12 einen verbreiterten Abschnitt 32a auf. Dieser hat einen größeren Durchmesser als der Zylindermantel 12, kann also nicht in diesen hineingefahren werden. Er besitzt zudem eine wannenförmige Ausgestaltung, wobei im Inneren dieser Wanne ein ebenfalls verbreiterter oberer Abschnitt 30c des Zylinderkolbens 30 aufgenommen wird. Der Antrieb der Zylinderhülse 32 greift an diesem Abschnitt 32a an. Die Zylinderhülse 32 muss gegenüber dem Zylinderkolben 30 lediglich zur Beaufschlagung des Dichtungspaketes 34 zwecks hermetischer Abdichtung verfahrbar sein, kann sich ansonsten jedoch gemeinsam mit dem Zylinderkolben bewegen.

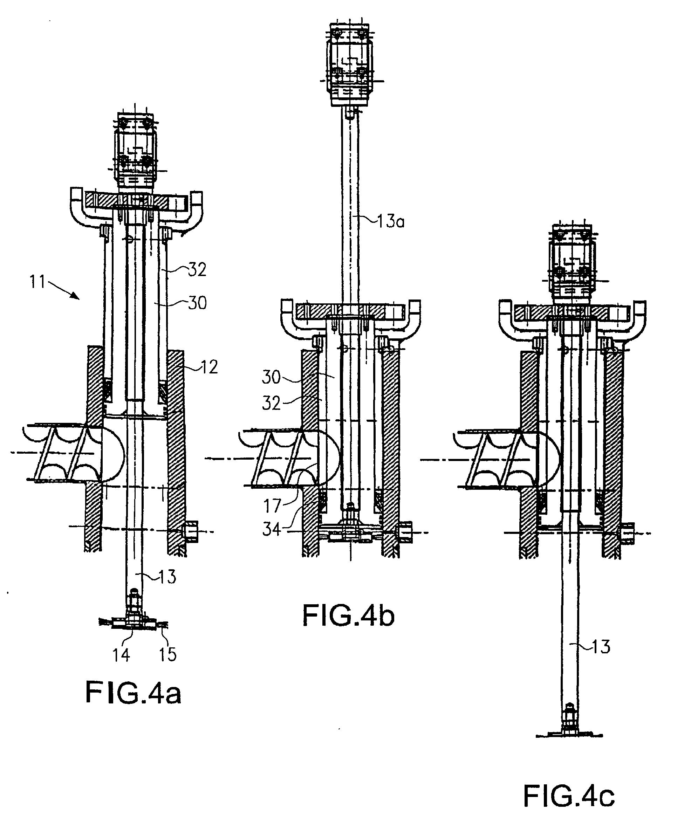

Das Zusammenspiel der beweglichen Komponenten 13, 30 und 32 des Verdichters 11 wird im Einzelnen bei der Beschreibung der Funktionsabläufe der Vorrichtung 1 mittels der Figuren 4 a) bis c) erläutert.

Im unteren Abschnitt des vertikalen Zylindermantels 12 befindet sich ein Bearbeitungsraum 20. In diesem Bearbeitungsraum, an dessen Einlass 20a der Verdichtungshub des Ausstoß-Verdichterkolbens 13 endet, wird der Abfall durch den Verdichter 11 verdichtet und anschließend durch die Bearbeitungseinrichtung 21 sterilisiert, die hier beispielsweise einen Mikrowellenstrahler aufweist. Die Sterilisierung durch die Bearbeitungseinrichtung 21 erfasst dabei das gesamte Volumen des Bearbeitungsraums 20, der von seinem Einlass 20a, seinem Boden 20b und den Wänden des Zylinders 12 begrenzt wird. Während der Sterilisierung können sich der untere Abschnitt 30a des Zylinderkolbens 30 und/oder der Verdichtungsteller 14 des Verdichterkolbens 13 am Einlass 20a des Bearbeitungsraums 20 befinden, um an der Sterilisierung teilzunehmen. Durch das Beaufschlagen des Dichtungspakets 34 durch die Zylinderhülse 32 ist während der Sterilisierung eine hermetische Abdichtung des Bearbeitungsraums 20 gewährleistet.

Am Einlass 20a des Bearbeitungsraums 20 ist ein Sensor 22 angeordnet, der wie der Sensor 16 das Vorhandensein oder den Durchgang von Abfall auf seiner Höhe registriert und mit einer geeigneten Auswerteeinheit verbunden ist (nicht gezeigt). Durch diesen Sensor wird ermittelt, wann der Bearbeitungsraum 20 so weit gefüllt ist, dass ein Verdichtungsvorgang des Verdichters 11 durchgeführt werden sollte.

Am Boden 20b des Bearbeitungsraums 20 befindet sich ein Auslass 23. Dieser Auslass 23 ist mittels eines Verschlusses 24 verschlossen, hier gebildet durch ein Kugelventil mit einem eigenen Antrieb 25. Dieser Verschluss wird nach einer ausreichenden Sterilisierung des Abfall durch die Bearbeitungseinrichtung 21 geöffnet; der Bearbeitungspfad des Abfalls durch die Vorrichtung 1 endet also hier.

Fig. 2 zeigt einen Vertikalschnitt durch den als Schnecke ausgebildete Förderer 8 in dessen Anfangsbereich 8a, orthogonal zu dem Schnitt in Fig. 1 an der in Fig. 1 gezeigten Stelle AA'. Insbesondere zeigt Fig. 2 die Seitenwände 4,5 des Einfülltrichters 3, die in einem nach oben offenen Winkel zueinander stehen. Dieser Winkel verringert sich in der Förderrichtung der Schnecke 8 von etwa 90° am Anfang, d.h. an der Stirnwand 6 (vgl. Fig. 1), auf etwa 45° am Ende, d.h. an der Stirnwand 7 (vgl. Fig. 1). Die Seitenwände 4,5 des Einfülltrichters 3 bilden in diesem Ausführungsbeispiel gleichzeitig die Seitenwände des Eingaberaums 2.

Der Boden 2a des Eingaberaums 2 schließt über einen gewissen Bereich seines Querschnitts über die gesamte Länge des Eingaberaums 2 bündig mit der Schnecke 8 ab; die Schnecke 8 liegt also teilweise innerhalb des Eingaberaums 2.
Oberhalb des Förderers 8 befindet sich im Eingaberaum 2 ein Sensor 16, der das Vorhandensein oder den Durchgang von Abfall auf seiner Höhe registriert und mit einer geeigneten Auswerteeinheit verbunden ist (nicht gezeigt), die die Bestimmung der seit der letzten Registrierung von Abfall verstrichenen Zeitspanne erlaubt.

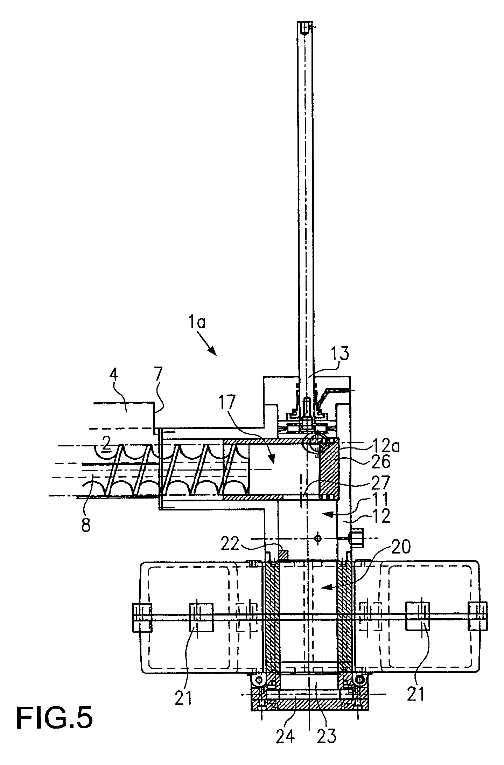

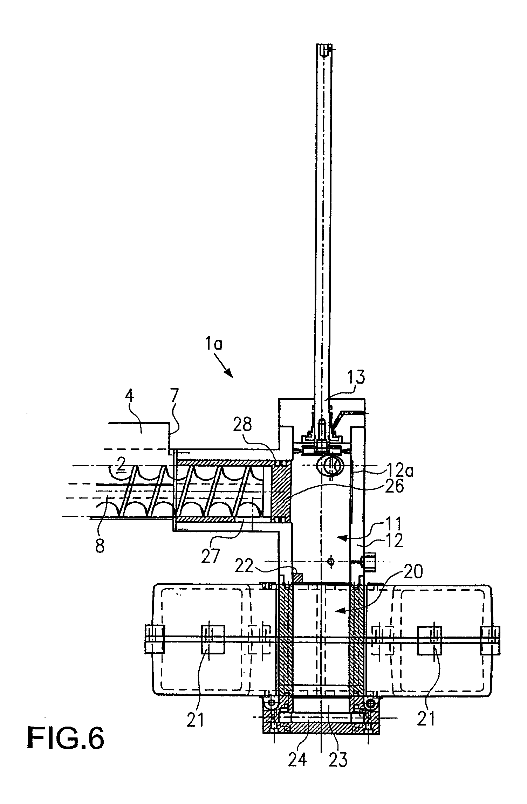

Die Fig. 5 und 6 zeigen einen Vertikalschnitt durch einen Ausschnitt eines zweiten Ausführungsbeispiels einer erfindungsgemäßen Vorrichtung 1a. Dieses Ausführungsbeispiel 1a entspricht dem in Fig. 1 gezeigten ersten Ausführungsbeispiel 1 bis auf die im Folgenden beschriebenen Eigenheiten; für identische Teile wurden jeweils identische Bezugszeichen verwendet.

Die Vorrichtung 1 a weist als Absperrhülse und als Abdichtung des Bearbeitungsraums 20 speziell eine Schiebehülse 26 auf, die an der Austrittsöffnung 17 des Förderers 8 bündig in dessen Gehäuse gelagert ist, und die auf ihrer Unterseite eine Durchgangsöffnung 27 besitzt. In Fig. 5 ist die Schiebehülse 26 in ihrer Durchgangsstellung gezeigt. Dabei ragt sie so weit in den Zylinder 12 des Verdichters 11 hinein, dass die Durchgangsöffnung 27 auf ihrer Unterseite frei liegt und den Durchgang des Abfalls vom Förderer 8 in den Verdichter 11 gestattet. Der Förderer 8 reicht in dieser Vorrichtung 1a nicht ganz bis an den Innenraum des Verdichters 11 heran, um in der Austrittsöffnung 17 Platz für die Aufnahme der Schiebehülse 26 in ihrer Sperrstellung (vgl. Fig. 6) zu lassen.

Wegen dieser Ausgestaltung der Absperrung ist auch der Verdichter 11 anders ausgebildet: Da die Funktion der mechanischen Absperrung bereits durch die Schiebehülse 26 erfüllt ist, kann bei diesem Ausführungsbeispiel auf die Zylinderhülse und den Zylinderkolben verzichtet werden. Als einziges im Zylindermantel 12 bewegliches Element bleibt demnach der Ausstoß-Verdichterkolben 13.

Zudem weist der Verdichter 11 in seiner Innenwand gegenüber der Austrittsöffnung 17 des Förderers eine Vertiefung 12a auf, die mit der Schiebehülse 26 fluchtet und deren Form und Durchmesser exakt dem Durchmesser und dem Querschnitt der Schiebehülse entsprechen. Diese Vertiefung 12a dient zur Aufnahme eines Abschnittes der Schiebehülse 26 in deren Durchgangsstellung. Ist die Schiebehülse 26 in die Vertiefung eingefahren, so können durch die Umlenkung des Abfalls auf die Schiebehülse 26 wirkende Rückstoßkräfte auf die Wand 12 des Verdichters 11 übertragen und von diesem aufgenommen werden. Damit wird die Belastung der Schiebhülse 26, insbesondere ihrer Lagerung, deutlich vermindert.

Als weiteren Unterschied zu dem in Fig. 1 gezeigten Beispiel 1 hat dieses Ausführungsbeispiel 1a ein Schiebeventil 28 statt eines Kugelventils als Verschluss 24 des Auslasses 23.

Fig. 6 zeigt denselben Ausschnitt desselben Ausführungsbeispiels 1a wie in Fig. 5. Im Unterschied zu der Situation in Fig. 5 befindet sich hier jedoch die Schiebehülse 26 in ihrer Sperrstellung. Dabei ist sie so weit gegenüber dem Förderer 8 verschoben, dass sie nicht mehr in den Zylindermantel 12 des Verdichters 11 hineinragt und damit die Bewegung des Verdichterkolbens 13 nicht mehr behindert. Dies wird dadurch ermöglicht, dass der Förderer 8 nicht ganz bis an den Innenraum des Verdichters 11 heranreicht, um in der Austrittsöffnung 17 Platz für die Aufnahme der Schiebehülse 26 zu lassen. In dieser Sperrstellung kann die Schiebehülse 26 durch einen geeigneten Mechanismus 28 verriegelt werden, so dass sie nicht unbeabsichtigt in den Zylinder 12 hineingedrückt werden kann; sie bildet dort die Abdichtung des Bearbeitungsraums 20 gegenüber der Fördereinrichtung 8. Diese Abdichtung kann dadurch noch verbessert werden, dass die Schiebehülse 26 in ihrer Sperrstellung ein Dichtungspaket (nicht gezeigt) so beaufschlagt, dass dieses eine hermetische Abdichtung bildet.
Der Ausstoß-Verdichterkolben 13 befindet sich in den Fig. 5 und 6 in seiner Bereitschaftsposition oberhalb der Öffnung 17 des Förderers 8, also vor oder nach einem Verdichtungshub.

Während des Verschiebens der Schiebehülse 26 von ihrer Durchgangsstellung (Fig. 5) in ihre Sperrstellung (Fig. 6) oder davor hat zweckmäßigerweise eine Umkehrung der Bewegungsrichtung des Förderers 8 stattgefunden, um den Endbereich des Förderers 8 von Abfall zu befreien und so eine leichtere Bewegung der Schiebehülse 26 zu ermöglichen. Bei einer Ausführung der Vorrichtung 1a mit solch einer Schiebehülse 26 ist dazu vorteilhafterweise der nicht vom Eingaberaum 2 überdeckte Anfangsbereich 8a des Förderers 8 (hier nicht gezeigt, vgl. Fig. 1) länger als die Strecke, um die die Schiebehülse 26 zwischen ihrer Durchgangs- und ihrer Sperrstellung verschoben werden muss.

Im üblichen Betrieb einer erfindungsgemäßen Vorrichtung 1,1a zum Bearbeiten von medizinischem Abfall fällt der bereits zerkleinerte Abfall in den Einfülltrichter 3 hinein, und damit in den Eingaberaum 2. Ein Sensor 16 im Eingaberaum registriert das Vorhandensein von Abfall und setzt den Antrieb 9 des Förderers 8 in Betrieb. Im Eingaberaum 2 ergreift der Förderer 8 den Abfall. Ist dieser Förderer 8 als eine Schnecke ausgebildet, so zwingt die Schnecke 8 den Abfall durch ihre Drehung in ihre Windungen und transportiert ihn in ihrer Förderrichtung, d.h. zu ihrer Austrittsöffnung 17 in den Verdichter 11. Durch die Drehung wird der Abfall aktiv in die Schnecke 8 hineingezogen. Durch das Hineinziehen immer neuen Abfalls erfolgt zudem eine leichte Vor-Verdichtung des Abfalls.

Sämtliche beweglichen Elemente des Verdichters 11, d.h. der Ausstoß-Verdichterkolben 13, sowie ggf. der Zylinderkolben 30 und die Zylinderhülse 32, befinden sich in ihrer Bereitschaftsposition oberhalb der Austrittsöffnung 17. Der vom Förderer 8 durch diese Öffnung geförderte Abfall fällt folglich unterhalb der beweglichen Elemente 13, 30, 32 in den vertikalen Zylindermantel 12 hinein und sammelt sich auf dem Boden 20b des Bearbeitungsraums 20 im unteren Bereich des Zylindermantels 12.
Sobald der Sensor 22 am oder oberhalb des Einlasses 20a des Bearbeitungsraums 20 eine permanente Anwesenheit von Abfall auf seiner Höhe feststellt, löst er den Verdichtungsvorgang aus. Dazu wird zunächst der Antrieb 9 des Förderers 8 gestoppt und anschließend der im Förderer 8 enthaltene Abfall zurück gefördert, bis ein gewisser Bereich vor der Austrittsöffnung 17 weitgehend frei von Abfall ist.

In den Figuren 4 a) bis c) ist dargestellt, wie der Verdichtungsvorgang bei dem ersten Ausführungsbeispiel, d.h. der Vorrichtung 1, abläuft. Zunächst wird allein der Ausstoß-Verdichterkolben 13 nach unten gefahren, um einen ersten Verdichtungshub auf den Abfall durchzuführen - bis zu der in Fig. 4 a) gezeigten Verdichtungsposition. Dabei streicht die Bürste 15 an der Innenseite des Zylindermantels 12 entlang und nimmt daran haftengebliebene Abfallreste mit.
Anschließend fährt der Ausstoß-Verdichterkolben 13 wieder nach oben in seine Bereitschaftsposition zurück, um von dort aus einen Haupt-Verdichtungshub gemeinsam mit dem Zylinderkolben 30 und der Zylinderhülse 32 auszuführen. Wegen der großen gemeinsamen Masse der drei Elemente und der Leistung ihrer Antriebe führt dieser Haupt-Verdichtungshub zu einer stärkeren Verdichtung des Abfalls als der erste Verdichtungshub. Wie in Fig. 4 b) dargestellt, erreichen die drei beweglichen Elemente 13, 30, 32 schließlich eine Position, bei der die Austrittsöffnung 17 vollständig von der Zylinderhülse 32 verschlossen ist. Durch das Eigengewicht der Zylinderhülse 32 oder durch die Leistung ihres Antriebs beaufschlagt die Zylinderhülse 32 in dieser Stellung das Dichtungspaket 34 derart, dass dieses sich verformt, sich der Innenwand des Zylindermantels 12 anpasst und somit eine hermetische Abdichtung des Bearbeitungsraums 20 vom oberen Bereich des Verdichters 11 bildet.
Hat der Sensor 22 registriert, dass sich der Ausstoß-Verdichterkolben 13 am Einlass 20a des Bearbeitungsraums 20 befindet, so bleiben die drei Komponenten 13, 30 und 32 in dieser Verdichtungsposition als Verschluss des Zylinders 12, während die Bearbeitungseinrichtung 21, z.B. ein Mikrowellenstrahler, den Abfall sterilisiert. Dabei wird gleichzeitig die Unterseite des Ausstoß-Verdichterkolbens 13 mit sterilisiert, insbesondere sein Verdichtungsteller 14. Bei dieser Sterilisierung werden im Bearbeitungsraum Temperaturen von über 150°C, typischerweise etwa 165°C, und Drücke von typischerweise 6,5 bar erreicht.

Im Anschluss an die erfolgte Sterilisierung wird der Verschluss 24 des Auslasses 23 der Vorrichtung 1 (oder 1a) geöffnet, damit der Abfall die Vorrichtung verlassen kann. Ist der Verschluss 24 beispielsweise als Kugelventil ausgebildet, so wird dieses durch seinen Antrieb 25 gedreht, bis seine Bohrung den Auslass 23 vom Bearbeitungsraum 20 nach außen freigibt. Ist der Verschluss 24 als Schiebeventil ausgebildet, so wird dieses entsprechend durch seinen Antrieb 25 zur Freigabe des Auslasses 23 verschoben.
Der Ausstoß-Verdichterkolben 13 wird nun zu einem zusätzlichen Hub nach unten ausgefahren, wie in Fig. 4 c) dargestellt, um den sterilisierten Abfall durch den Auslass 23 aus der Vorrichtung auszustoßen. Eventuell sind dazu mehrere einzelne Hübe durchführbar, um den Abfall in kleineren Einheiten auszuschieben. Insgesamt führt der Ausstoß-Verdichterkolben 13 bei diesem Ausschieben einen zusätzlichen Hub bis zur Höhe des Bodens 20b des Bearbeitungsraumes 20 aus, d.h. der zusätzliche Hub entspricht der Höhe des Bearbeitungsraumes 20.

Nach dem Ausschieben des Abfalls wird der Verschluss 24 wieder verschlossen. Die Zylinderhülse 32 wird so gegen den Zylinderkolben 30 verfahren, dass die auf das Dichtungspaket 34 ausgeübte Kraft nachlässt. Anschließend fahren der Ausstoß-Verdichterkolben 13, der Zylinderkolben 30 und die Zylinderhülse 32 in ihre Bereitschaftsposition. Sofern der Sensor 16 noch die Anwesenheit von Abfall im Eingaberaum 2 feststellt oder seine letzte Registrierung von Abfall noch nicht genügend lange zurückliegt, wird der Antrieb 9 des Förderers 8 wieder in Gang gesetzt, und der gesamte Vorgang beginnt von Neuem. "Genügend lange" bedeutet dabei, dass der Förderer 8 so lange fördert, bis sie mit hoher Wahrscheinlichkeit frei von Abfall ist.

Bei der in Fig. 5 und 6 dargestellten Vorrichtung 1a, d.h. dem zweiten Ausführungsbeispiel, wird vor dem Verdichtungsvorgang zunächst die Schiebehülse 26 wird aus ihrer Durchgangsstellung in die Sperrstellung verschoben, so dass die Durchgangsöffnung 27 auf ihrer Unterseite verschlossen ist. Damit ist die Austrittsöffnung 17 mechanisch verschlossen, gleichzeitig bildet die Schiebehülse 26 die Abdichtung des Bearbeitungsraums 20. Ggf. wird die Schiebehülse 26 in ihrer Sperrstellung verriegelt.
Nun führt der Ausstoß-Verdichterkolben 13 einen oder mehrere Verdichtungshübe auf den im Bearbeitungsraum 20 enthaltenen Abfall aus.
Nach der Sterilisierung wird der Ausstoß-Verdichterkolben 13 in seine Bereitschaftsposition oberhalb der Austrittöffnung 17 gefahren; anschließend wird die Schiebehülse 26 wieder in ihre Durchgangsstellung gebracht, d.h. in die Vertiefung 12a in der Wand des Zylindermantels 12 gefahren.

Vor einem Abschalten der Vorrichtung kann die Absperrhülse (26, 32) in ihre Sperrstellung gebracht werden, so dass auch bei abgeschalteter Vorrichtung das Innere der Vorrichtung, insbesondere der Verdichter, abgesperrt ist.

Die Erfindung beschränkt sich nicht auf die beschriebenen Ausführungsbeispiele, sondern es sind eine Vielzahl weiterer Ausführungsbeispiele denkbar. Insbesondere kann z.B. die Zahl der unter einem Winkel zueinander wirkenden, nacheinander entlang des Verarbeitungspfades durchlaufenen Verdichter auf zwei oder mehr erhöht werden. Ferner kann der Verdichter statt eines Kolbens einen andersartigen, stoßartigen Verdichter aufweisen. Der oder die Sensoren zur Kontrolle des Füllstandes im Verdichter können wahlweise entfallen und z.B. über eine Messung der Leistungskurve des Antriebs des Verdichters ersetzt werden. Sobald die Leistung dieses Antriebs einen festzulegenden Schwellwert überschreitet, gilt dies als Maß für eine erfolgte Verdichtung und kann als Information über die Füllhöhe herangezogen werden.

## Patentansprüche

1. Vorrichtung (1) zum Bearbeiten von medizinischem Abfall, mit einem Eingaberaum (2), einem Förderer zum Transport des Abfalls (8) zu einem Verdichter (11), mindestens einer sterilisierenden Bearbeitungseinrichtung (21) und einem Auslass (23) zum Auslassen des sterilisierten Abfalls,
**dadurch gekennzeichnet,**
**dass** sie eine verfahrbare Absperrhülse (26, 32) zwischen dem Förderer (8) und dem Verdichter (11) aufweist.

2. Vorrichtung nach Anspruch 1,
**dadurch gekennzeichnet,**
**dass** die Absperrhülse als gegenüber dem Förderer (8) verschiebbare Schiebehülse (26) um das Ende des Förderers (8) ausgebildet ist.

3. Vorrichtung nach Anspruch 2,
**dadurch gekennzeichnet,**
**dass** die Schiebehülse (26) mindestens zwei unterschiedliche Stellungen hat, namentlich eine Durchgangsstellung und eine Sperrstellung, sowie eine Durchgangsöffnung (27) zum Durchführen des vom ersten Verdichter (8) beförderten Abfalls in den Verdichter (11), wobei diese Durchgangsöffnung (27) in der Durchgangsstellung geöffnet und in der Sperrstellung gesperrt ist.

4. Vorrichtung nach Anspruch 1,
**dadurch gekennzeichnet,**
**dass** die Absperrhülse (32) im Verdichter (11) geführt und so ausgebildet und angeordnet ist, dass sie als Verschluss einer Austrittsöffnung (17) dem Förderer (8) gegenüber dem Verdichter (11) dient.

5. Vorrichtung nach wenigstens einem der vorangehenden Ansprüche,
**dadurch gekennzeichnet,**
**dass** der Verdichter (11) einen in einem Zylinder (12) geführten Ausstoß-Verdichterkolben (13) aufweist.

6. Vorrichtung nach wenigstens einem der vorangehenden Ansprüche,
**dadurch gekennzeichnet,**
**dass** die Achse des Zylinders (12) des Verdichters (11) vertikal angeordnet ist.

7. Vorrichtung nach Anspruch wenigstens einem der vorangehenden Ansprüche,
**dadurch gekennzeichnet,**
**dass** der Verdichter (11) zusätzlich zum Ausstoß-Verdichterkolben (13) als weitere im Zylinder (12) bewegbare Elemente mindestens einen Zylinderkolben (30) und die Absperrhülse (32) aufweist.

8. Vorrichtung nach wenigstens einem der vorangehenden Ansprüche,
**dadurch gekennzeichnet,**
**dass** ein Dichtungspaket (34) am Zylinderkolben (30) oder an der Absperrhülse (26, 32) angeordnet ist, welches von der Absperrhülse (26, 32) zur Abdichtung beaufschlagbar ist.

9. Vorrichtung nach wenigstens einem der vorangehenden Ansprüche,
**dadurch gekennzeichnet,**
**dass** sie im Eingaberaum (2) einen Sensor (16) zur Registrierung der Eingabe oder der Anwesenheit von Abfall aufweist.

10. Vorrichtung nach wenigstens einem der vorangehenden Ansprüche,
**dadurch gekennzeichnet,**
**dass** der Sensor (16) als Lichtschranke oberhalb des Förderers (8) ausgebildet ist.

11. Vorrichtung nach wenigstens einem der vorangehenden Ansprüche,
**dadurch gekennzeichnet,**
**dass** der Sensor (16) mit einer Auswerteeinheit zum Bestimmen der seit der letzten Registrierung von Abfall verstrichenen Zeit verbunden ist.

12. Vorrichtung nach wenigstens einem der vorangehenden Ansprüche,
**dadurch gekennzeichnet,**
**dass** der Förderer (8) einen in seiner Arbeitsrichtung umschaltbaren Antrieb (9) aufweist.

13. Vorrichtung nach wenigstens einem der vorangehenden Ansprüche,
**dadurch gekennzeichnet,**
**dass** der Anfangsbereich (8a) des Förderers (8) außerhalb des Eingaberaums (2) liegt.

14. Vorrichtung nach wenigstens einem der vorangehenden Ansprüche,
**dadurch gekennzeichnet,**
**dass** im Eingaberaum (2) und/oder im Förderer (8), insbesondere in deren Anfangsbereich (8a), eine oder mehrere Vorrichtungen zum Besprühen des Abfall mit einer Desinfektionslösung vorgesehen sind.

15. Vorrichtung nach wenigstens einem der vorangehenden Ansprüche,
**dadurch gekennzeichnet,**
**dass** der Hub des Ausstoß-Verdichterkolbens (13) in etwa am Auslass (23) der Vorrichtung endet.

16. Vorrichtung nach wenigstens einem der vorangehenden Ansprüche,
**dadurch gekennzeichnet,**
**dass** der Hub des Ausstoß-Verdichterkolbens (13) so steuerbar ist, dass die Unterseite und/oder ein unterer Abschnitt des Ausstoß-Verdichterkolbens (13) während der Sterilisierung selbst dieser Sterilisierung ausgesetzt ist.

17. Vorrichtung nach wenigstens einem der vorangehenden Ansprüche,
**dadurch gekennzeichnet,**
**dass** die Antriebe des Förderers (8) und/oder der bewegbaren Elemente des Verdichters (13, 30) und/oder der Absperrhülse (26, 32) in ihrer Bewegung miteinander koordiniert sind.

18. Vorrichtung nach wenigstens einem der vorangehenden Ansprüche,
**dadurch gekennzeichnet,**
**dass** sich im Verdichter (11) mindestens ein Sensor (22) befindet, der die Anwesenheit von Abfall an seiner Position innerhalb des Verdichters (11) registriert.

19. Vorrichtung nach wenigstens einem der Ansprüche 8 bis 16,
**dadurch gekennzeichnet,**
**dass** die vorhandenen Sensoren (16, 22) mit den Antrieben des Förderers (8) und/oder der bewegbaren Komponenten des Verdichters (13, 30, 32) und/oder der Absperrhülse (26) koordiniert sind.

20. Vorrichtung nach wenigstens einem der vorangehenden Ansprüche,
**dadurch gekennzeichnet,**
**dass** die sterilisierende Bearbeitungseinrichtung (21) einen Mikrowellenstrahler aufweist.

21. Vorrichtung nach wenigstens einem der vorangehenden Ansprüche,
**dadurch gekennzeichnet,**
**dass** der Auslass (23) einen Verschluss (24) aufweist, insbesondere ein Kugelventil oder ein Schiebeventil.
